# EUROPEAN PATENT SPECIFICATION

(11) **EP 4 205 801 B1**
(45) Date of publication and mention of the grant of the patent: **24.07.2024**
(21) Application number: 22217123.3
(22) Date of filing: 29.12.2022
(51) Int. Cl.: A61N 1/05, A61N 1/36

(54) **DEVICES AND METHODS FOR BAROREFLEX ACTIVATION**
VORRICHTUNGEN UND VERFAHREN ZUR BAROREFLEX-AKTIVIERUNG
DISPOSITIFS ET PROCÉDÉS D'ACTIVATION BARORÉFLEXE

(30) Priority: 29.12.2021 US 202163294649 P
(43) Date of publication of application: 05.07.2023
(60) Divisional application: 24183118.9
(73) Proprietor: CVRx, Inc., Minneapolis, MN 55445 (US)
(72) Inventor: PARKER, Luke, Minneapolis, 55445 (US); SUNDBERG, Gregory, Minneapolis, 55445 (US); PIGNATO, Paul, Minneapolis, 55445 (US)
(74) Representative: Clark, Jane Anne

(56) References cited:
- US-A1- 2013 110 208
- US-A1- 2014 257 363
- US-A1- 2020 315 700
- US-B1- 9 216 280

## Description

### TECHNICAL FIELD

The present disclosure relates generally to surgical implant methods and devices, and more particularly the present disclosure relates to improved minimally invasive methods and devices for implanting one or more components of a baroreflex activation device.

### BACKGROUND

Cardiovascular disease is a major contributor to patient illness and mortality and is also a primary driver of health care expenditure. Heart failure is the final common expression of a variety of cardiovascular disorders, characterized by an inability of the heart to pump enough blood to meet a patient's needs. Symptoms of heart failure include fatigue, reduced exercise capacity and shortness of breath.

Hypertension, or high blood pressure, is a major cardiovascular disorder that is estimated to affect tens of millions of people in the United Sates alone. Hypertension is a leading cause of heart failure and stroke, is the primary cause of death for tens of thousands of patients per year, and is listed as a primary or contributing cause of death for hundreds of thousands of patients per year in the United States alone.

Accordingly, heart failure and hypertension are serious health problems demanding significant research and development for the treatment thereof.

One approach for treating hypertension and/or heart failure is baroreflex activation therapy (or "BAT"), which comprises stimulation of baroreceptors and/or associated nerves or nerve structures of a patient. Baroreceptors are sensory nerve ends that are profusely distributed within the walls of the major arteries, as well in the heart, aortic arch, carotid sinus or arteries, and in the low-pressure side of the vasculature such as the pulmonary artery and vena cava. Baroreceptor signals are used to activate a number of body systems which collectively may be referred to as the baroreflex system. Baroreceptors are connected to the brain via the nervous system. Thus, the brain is able to detect changes in blood pressure, which can be related to, or indicative of, cardiac output.

Baroreflex activation therapy works by artificially activating the carotid sinus baroreflex. U.S. Patent No. 6,522,926 to Kieval, et al. discloses baroreflex activation devices, systems and methods for activating baroreceptors to regulate blood pressure for the treatment of hypertension and/or heart failure (to counteract the above-described pressor response). Generally speaking, the baroreflex activation device may be activated, deactivated or otherwise modulated to activate one or more baroreceptors and induce a baroreceptor signal or a change in the baroreceptor signal to thereby affect a change in the baroreflex system. The baroreflex activation device may be activated, deactivated, or otherwise modulated continuously, periodically, or episodically. The baroreflex activation device may utilize electrical, mechanical, thermal, chemical, or biological means, or a combination thereof to activate the baroreceptor. Baroreceptors of the patient may be activated directly, or activated indirectly for example via the adjacent vascular tissue.

Activating the baroreflex of a patient (by, for example, stimulation of baroreceptors in the aortic arch or carotid sinus or by stimulating the nerve emanating from baroreceptors such as the aortic nerve, carotid sinus nerve or vagus nerve) increases afferent electrical signals. For example, through the carotid sinus nerve (Hering's nerve, a branch of the glossopharyngeal nerve, cranial nerve IX) or aortic nerve (a branch of the vagus nerve, cranial nerve X) to the medullary brain centers that regulate autonomic tone. Increased afferent signals to these medullary centers cause a reduction in sympathetic tone and an increase in parasympathetic tone. This results in lower heart rate, reduced sodium and water reabsorption by the kidney resulting in a diuresis, relaxation of the smooth muscle in the blood vessels which results in vasodilatation and a reduction in excessive blood pressure, cardiac workload and circulating neurohormone levels.

Thus, peripheral activation of the baroreflex results in a physiologic response whereby cardiovascular function is controlled by mechanisms determined by the integrative action of the central nervous system action on all peripheral organs and blood vessels. In hypertension clinical trials, BAT has been demonstrated to reduce excessive blood pressure. In heart failure clinical trials, BAT has been demonstrated to reduce patient symptoms, improve patient quality of life and functional capacity, improve cardiac function, decrease levels of circulating cardiac stress biomarkers, and reduce patient rehospitalization.

Early approaches to BAT systems and associated implant procedures typically required relatively large incisions on one or both sides of a patient's neck to create sufficient access to the target vasculature in the area of the carotid sinus. The carotid artery was dissected free, one or more electrode pads were wrapped around the artery and sutured in place.

Current generation implantable BAT devices and systems offer pulse generator housings and associated therapy electrodes with reduced form factors as compared to early systems. One such system, described in U.S. Patent No. 8,437,867 to Murney et al., includes an implantable pulse generator and associated circuitry contained within a hermetically sealed housing, an elongate flexible electrical lead connectable to the housing, and a monopolar electrode structure coupled with the electrical lead.

Current generation implantable BAT devices and systems also offer improvements to the implant procedure. Smaller electrode structures typically allow smaller incisions on the patient. The electrode structure described in the above-mentioned U.S. Patent No. 8,437,867 can be implanted via a minimally invasive approach, as described therein.

A recent improvement to the implant procedure for BAT devices and systems involves percutaneous, minimally invasive methods and related devices, as described in published PCT application WO 2020/037145 to Pignato et al. The percutaneous implant methods and devices described therein represent an improvement over prior approaches, however, opportunities exist for further refinements to fixation of the electrode, in lead body design, electrode design, and delivery tools and techniques.

US 2020/315700 A1 discloses an endoluminal nerve modulation device and methods for using thereof.

US 2013/110208 A1 discloses an electrostimulation system, and electrostimulation electrode assembly and biological implantable electrode thereof.

US 9216280 B1 discloses an endovascular electrode system for tissue stimulation. US 2014/257363 A1 discloses micro-fabricated embolic devices.

The present disclosure addresses these concerns.

### SUMMARY

Some embodiments of the present disclosure relate to minimally invasive implantation of one or more components of a baroreflex activation device or system. Improvements to the lead body, lead fixation, electrode fixation, delivery methods, or combinations are contemplated.

In an embodiment, the lead body may include a passive fixation structure molded onto or otherwise coupled with the lead body. The lead body may have polyurethane barbs molded directly onto the tubing, with the electrode cable. The barbs molded directly into the lead tubing may help prevent the lead from moving once the electrode and lead is placed.

In another embodiment, the lead body may include an active fixation arrangement movable between a delivery state and a deployed state. The lead body may include a plurality of barbs that are retracted in the delivery state, depicting a smooth lead with no interruptions. In the deployed state, the barbs may extend through angled cuts in the lead body. The lead body may be made of a rigid plastic or a bio-resorbable material on one side of the polymer barbed wire. The barbs may extend or bend outwards to create a rigid structure to resist movement of the lead body after implantation.

In another embodiment, the lead body may include multiple rows of active fixation elements arranged around a circumference of the lead body. For example, the lead may include three lumens disposed therein, each lumen containing barbed fixation elements configured to be movable between a delivery state and a deployed state. The barbs may be made of a low durometer polymer (e.g., below 100A, below 80A, or as desired) in order to more effectively puncture the wall of the lumen. In yet another embodiment, there may be any number of lumens included in the lead design. The barbs may extend during device assembly during the manufacturing stage, or the barbs may extend as a mechanism during the delivery of the baroreflex device, after implantation. The barbs may be exposed on the lead body as the polymer wires of the lumen design are pulled backwards by a surgeon after implantation.

In another embodiment, the electrode, backer, or electrode structure may be altered to assist in fixation. The electrode may have a coil backer molded into a backer. The coil backer may be made of nitinol, or may vary in material and structure. The coil backer may vary in height, and the number of coil revolutions. The coil backer may be combined with any combination of the barbed structures for lead body designs discussed above.

In another embodiment, the back side of the electrode may have nitinol or rigid polymer hooks. The height and number of hooks may vary, in order to optimize fixation and ease of delivery. The hooks may be arranged in synchronous rows of any number, on an electrode body. The hooks may be on more than one surface, or the hooks may vary in direction. The hooks may resist movement as the lead is removed after implantation, by gripping to tissue after the electrode has been placed.

In another embodiment, the electrode structure may have a wing or hood or domed structure to restrict backward movement of the lead. The wing or hood may be comprised of nitinol frames, or a molded polymer structure. The wing or hood may be combined with any combination of the barbed structures for lead body designs discussed above.

In another embodiment, the electrode body may have a modified tool or device to aid in delivery. The electrode body may have a needle head that is permanently, or removably attached to create a pathway to the targeted delivery site. The needle head may stay with the electrode at the therapy site, or there may be a method of detachment for the needle head to be removed with the lead after implantation. The needle head may protect the space above the targeted therapy site, while creating structure for fixation if it is not detached after delivery and implantation.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIG. 1 is a schematic illustration of a baroreceptor activation system according to an embodiment.
FIG. 2 is a top perspective view of a lead assembly with a molded disc-shaped electrode.
FIG. 3 is a bottom view of FIG. 2.
FIG. 4A is a perspective view of a lead assembly with passive fixation elements, according to an embodiment.
FIG. 4B is a perspective view of the passive fixation elements of FIG. 4A.
FIG. 5 is a perspective view of a barbed lead body with barbs extended, according to an embodiment.
FIG. 6A is a perspective view of a lead body having active fixation elements depicted in a delivery position, according to an embodiment.
FIGS. 6B-6D are views of a lead body having active fixation elements depicted in a deployed position.
FIG. 7A is a perspective view of a coil backer for an electrode structure, according to an embodiment.
Fig. 7B is a perspective view of an electrode structure including a coil backer and an associated lead, according to an embodiment.
FIG. 8 is a perspective view of an electrode structure including a plurality of passive fixation elements, according to an embodiment.
FIG. 9 is a perspective view of a hood or a poly-wing backer for an electrode.
FIG. 10A a perspective view of a needle tip for an electrode.
FIG. 10B is a rear view of a needle tip for an electrode.
FIG. 10C is a front view of a needle tip for an electrode.
FIG. 11 is a schematic depicting anatomical axes.

While various embodiments are amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the subject matter as defined by the claims.

### DETAILED DESCRIPTION OF THE DRAWINGS

The following detailed description should be read with reference to the drawings in which similar elements in different drawings are numbered the same. The drawings, which are not necessarily to scale, depict illustrative embodiments and are not intended to limit the scope of the disclosure.

For information pertaining to the cardiovascular, circulatory and nervous systems, as well as baroreceptor and baroreflex therapy systems that may be used in whole or in part with embodiments of the present disclosure, reference is made to the following commonly assigned published applications and patents: U.S. Published Patent Application Nos. 2006/0004417 to Rossing et al., 2006/0074453 to Kieval et al. and 2008/0082137 to Kieval et al., and U.S. Pat. Nos. 6,522,926 to Kieval et al., 6,850,801 to Kieval et al., 6,985,774 to Kieval et al., 7,480,532 to Kieval et al., 7,499,747 to Kieval et al., 7,835,797 to Rossing et al., 7,840,271 to Kieval et al., 8,086,314 to Kieval, 8,326,430 to Georgakopoulos et al., and 9,345,877 to Pignato et al..

Embodiments of the present disclosure generally pertain to improved devices and methods for implanting a baroreflex activation therapy system. In an embodiment, the baroreflex activation therapy system can be as described in U.S. Patent No. 8,437,867 to Murney et al., the disclosure of which is incorporated by reference herein. A description of percutaneous, minimally invasive methods and related devices, is included in published PCT application WO 2020/037145 ("the '145 PCT publication") to Pignato et al.

The '145 PCT publication generally describes a lead delivery tool which can be introduced over a guidewire, to deliver an electrode attached to a lead to a target implant location. The electrode and corresponding electrode structure may include passive fixation elements such as a mesh bundle or barbed suture, the passive fixation elements configured to retain the electrode and electrode structure in place at the target implant location. However, use of the lead delivery tool described in the '145 PCT publication dilates or "tunnels" soft tissue below the skin along the path of introduction, which includes the area proximate the target implant location where the electrode structure is implanted. In some instances, the space created by the dilation is large enough that the passive fixation elements included with the electrode structure can be less effective than desired in one or more directions, which can contribute to undesirable migration of the electrode structure, or reduced effectiveness of therapy delivered via the electrode, or both.

Embodiments of the present disclosure may provide more robust fixation of a lead body or electrode structure at a target implant location, less invasive implant procedures, combinations thereof, or other advantages. Where applicable, description of common components among embodiments of the present disclosure may not be repeated in the following, and like numerals may designate like parts throughout that are corresponding or analogous.

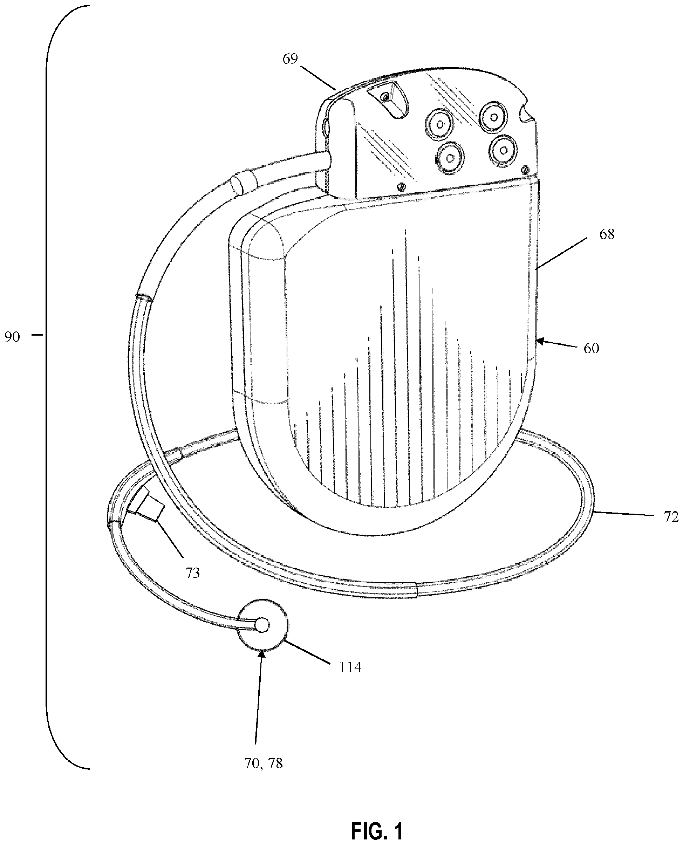

Referring now to FIG. 1, an embodiment of a baroreflex activation therapy system 90 is depicted, including a control system 60, a baroreflex activation device 70, and a lead 72. In embodiments, control system 60 may alternately be referred to as an implantable medical device, an implantable pulse generator, or the like. Control system 60 is understood to include the necessary circuitry and associated components for generating and delivering electrical therapy signals, and other functions. Control system 60 is configured to be implanted within a patient and includes a hermetically sealed housing 68, a header 69 adapted to facilitate connection of one or more leads 72, as well as a case electrode 67 on at least a portion of the outer surface of housing 68. Electrode 67 may also be referred to as an indifferent electrode, common electrode, or reference electrode. As used herein, the words "housing," "enclosure," "case," and "can" are synonymous when used to refer to housing 68.

In embodiments, baroreflex activation device 70 may comprise an electrode structure 78 which generally includes an electrode 112 mounted on, integrated with, or otherwise coupled to a backer 114 (with electrode 112 arranged on an underside of electrode structure 78 of FIG. 1 and therefore not depicted in FIG. 1). Backer 114 may also be understood as a backing layer, or simply the electrode structure body. Embodiments of backer 114 described herein may be constructed of flexible Dacron-reinforced insulated silicone, or a generally rigid polycarbonate or other polymer. In an embodiment, electrode structure 78 is considered to be part of lead 72. Electrode 112 may comprise platinum iridium, and may include a surface treatment, such as iridium oxide or titanium nitride and/or can include steroid, antiinflammatory, antibiotic and/or analgesic compounds, for example. Electrode structure 78 may comprise circular structures, or other suitable arrangements without departing from the spirit of the disclosure.

In one embodiment, electrode 112 may have a diameter of about 1 mm, and backer 114 may have a diameter of about 6mm. However, it is contemplated that electrode 112 may have a diameter within a range of about 0.25 mm-3 mm, while backer 114 may have a diameter within a range of about 1 mm-10 mm. In one embodiment the diameter of backer 114 is at least twice the diameter of electrode 112. In an embodiment, the surface area of the backer is at least twice the area of the electrode. In an embodiment, the surface area of the backer is about ten times the area of the electrode.

In embodiments, electrode 112 comprises a cathode, and in one embodiment housing 68 of control system 60 may comprise an anode. In another embodiment, reference electrode 67 may comprise an anode. In another embodiment, an anode may be provided as part of lead 72. In another embodiment, an anode is provided on a second lead which is also coupled to control system 60. In embodiments, the anode may be sufficiently larger than the cathode, for example ten times larger. In another embodiment the anode is fifty times larger than the cathode. Further, the anode and cathode are preferably positioned at a minimum distance away from one another, for example, the distance may be about twenty times the cathode diameter. In another embodiment, the distance between anode and cathode is at least fifty times the cathode diameter.

One or more electrode structures 78 may be provided as part of a baroreflex activation therapy system according to embodiments. For example, a first electrode structure 78 may be positioned at a first anatomical location, while a second electrode structure 78 is positioned at a second anatomical location, such as for example at a left carotid sinus and a right carotid sinus. Or a first electrode structure 78 may be positioned at a first anatomical location while a second electrode structure 78 is positioned at a second anatomical location proximate the first anatomical location, such as for example positioning first and second electrode structures proximate one another on the left carotid sinus and/or carotid arteries.

Lead 72 generally includes a proximal end having a connector for selectively coupling to header 69 of pulse generator housing 68, a body portion 75, and a distal end. In one embodiment, baroreceptor activation device 70 is disposed on the distal end of lead 72, although it will be understood that other arrangements of baroreceptor activation device 70 and lead 72 are within the scope of the present disclosure. In embodiments, leads described herein may be referred to as lead assemblies. Leads described herein are understood to include a conductor disposed within the lead body, the conductor extending between and electrically coupling the electrode to the connector on the proximal end of the lead.

In embodiments, the conductor may be connected to electrode structure 78 by mechanical or thermal means. In an embodiment, the conductor may be constructed as a multifilament / stranded cable (e.g., 7x7) rather than the traditional wound coil configuration. In embodiments, cable construction of the conductor could have a diameter in the range of 0.127 mm to 6.35 mm (0.005 inches to 0.250 inches).

The control system 60 generates a control signal (also referred to as a therapy signal), which activates, deactivates or otherwise modulates the baroreflex activation device 70. In an embodiment, the therapy signal is in the range of about 1 to 10 volts, at a rate between 5 Hz and 200 Hz, with an amplitude between 1 and 50 milliamps. Typically, activation of the device 70 via the control signal results in activation of baroreceptors of a patient. Alternatively, deactivation or modulation of the baroreflex activation device 70 may cause or modify activation of the baroreceptors. The baroreflex activation device 70 may include a wide variety of devices which utilize electrical means, such as electrodes, to activate baroreceptors.

The control system memory may contain data related to the therapy signal, a sensor signal, and/or values and commands provided by an input device, such as an external programmer operated by a patient or a clinician. The memory may also include software containing one or more algorithms defining one or more functions or relationships between the therapy signal and the sensor signal. The algorithm may dictate activation or deactivation therapy signals depending on the sensor signal or a mathematical derivative thereof. The algorithm may dictate an activation or deactivation therapy signal when the sensor signal falls below a lower predetermined threshold value, rises above an upper predetermined threshold value or when the sensor signal indicates a specific physiologic event. The memory may also include software containing one or more algorithms for determining patient physiological parameters based on a measured parameter.

In embodiments, aspects of the disclosure pertain to improvements to the lead, the lead body, the electrode structure, or combinations thereof.

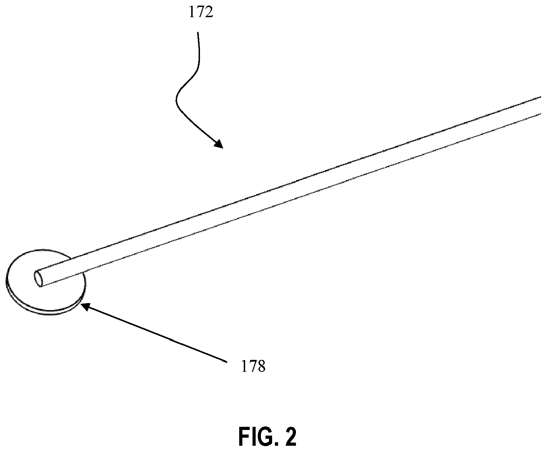

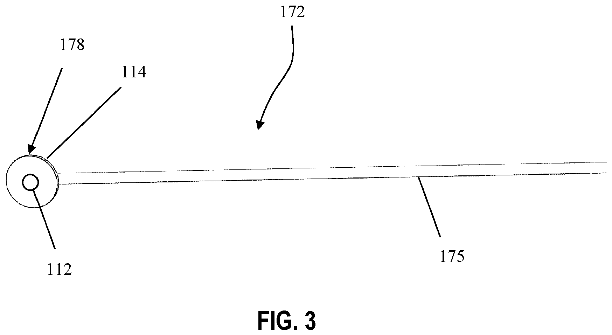

Referring now to FIGS. 2-3, a lead assembly 172 is depicted according to an embodiment of the disclosure. Lead assembly 172 includes a lead body 175, an electrode structure 178, and an electrode 112 mounted on, integrated with, or otherwise coupled to a backer 114. Electrode structure 178 may be constructed from a generally rigid, non-pliable material such as tecothane, silicone or nitinol.

Prior approaches to electrode structure designs, for not only baroreflex activation devices specifically but for implantable stimulation devices generally, favored highly flexible, compliant constructions. One common approach utilized silicone, with an optional reinforced nylon layer for resistance to tearing if the electrode structure was intended to be sutured at a target implant location. The use of a flexible, compliant electrode structure has been favored for, among other reasons, ability to readily conform to curved or irregular anatomical structures such as the outside (outer surface) of a blood vessel.

In contrast, the inventors of the present disclosure have found the ability for an electrode structure to conform to a curved anatomical structure less critical than previously thought. A generally rigid electrode structure, such as electrode structure 178 depicted in FIGS. 2-3, may provide advantages over prior solutions. In embodiments, a rigid electrode structure may be used, in combination with an appropriate delivery tool, for blunt dissection of tissue during an implant procedure. For example, a disc-shaped rigid electrode structure may be utilized such that a leading or distal edge of the circumference of the disc dissects tissue along a desired implant pathway. In embodiments, an introducer device or one or more needles may be initially used to create a dissected pathway prior to introduction of the electrode structure.

In other embodiments, aspects of the present disclosure pertain to improvements for fixation of the lead. Fixation may be of the passive type, active type, or combinations thereof.

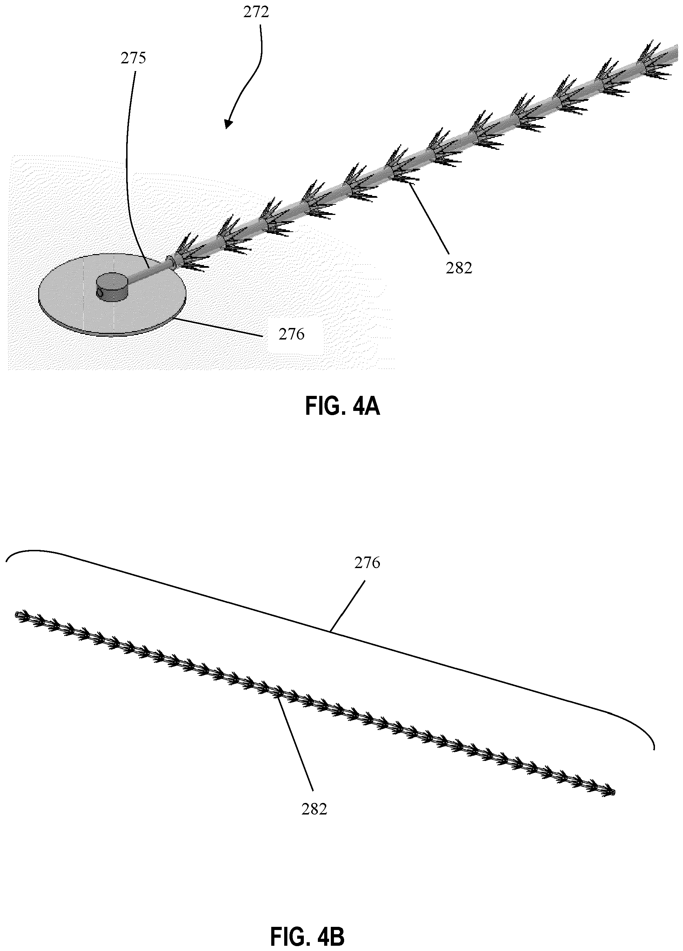

Referring now to FIGS. 4A-4B, a lead assembly 272 is depicted according to an embodiment of the disclosure. Lead assembly 272 includes a lead body 275, an electrode structure 278, and fixation tubing 276. Fixation tubing 276 includes one or more passive fixation elements arranged thereon, such as barbs 282. The fixation tubing 276 may extend over the length of the lead body 275, or the fixation tubing 276 may extend only over a distal portion of the lead body 275. In embodiments, barbs 282 may be molded in an outward, deployed configuration as pictured. In additional embodiments, barbs 282 may be molded in alternative configurations to aid in fixation after lead 272 placement. In embodiments, a length of the barb may be between 0.5 and 2.5 times a radius of lead body 275. In embodiments, barbs 282 may extend along only a portion of lead body 275. In embodiments, barbs 282 may extend along an entire length of lead body 275. The barbs 282 may be extended during device assembly during the manufacturing stage, or the barbs 282 may extend as a mechanism during the delivery of the baroreflex activation device 70, after implantation. The barbs 282 may be exposed directly on lead assembly 272 or on fixation tubing 276. In embodiments, barbs 282 may be constructed of polyurethane, rigid polymer, or a bioresorbable material. In embodiments, barbs 282 may be constructed of nitinol or other suitable materials. In embodiments, barbs 282 may be constructed from a relatively flexible material.

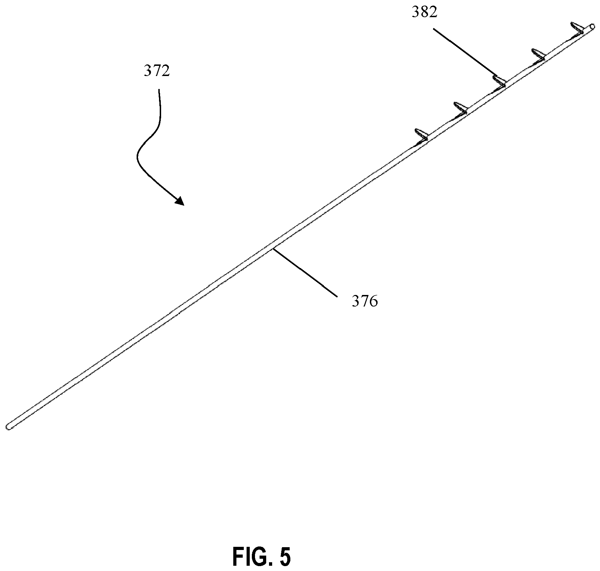

Referring now to Fig. 5, a lead assembly 372 is depicted according to an embodiment of the disclosure. Lead assembly 372 including a fixation arrangement which may be included on a length of tubing 376 coupleable to a lead body, or may be included directly as part of a lead body (not shown). A plurality of barbs 382 are included and can be generally arranged in a row along a length of tubing 376 or a lead body. Barbs 382 are movable between a retracted position (not shown) and the deployed, extended position depicted in Fig. 5. In an embodiment, barbs 282 may be biased in a deployed configuration, and an introducer sheath may be provided over at least a portion of the lead body such that the sheath retains barbs 282 in the retracted position, and removal of the sheath permits barbs 282 to automatically be moved to the deployed position. In other embodiments, barbs 282 may be manually movable between the retracted and deployed positions, for example with the use of a stylet or other tool to manipulate a mechanism within a lumen of lead assembly 372 (such as a lead screw).

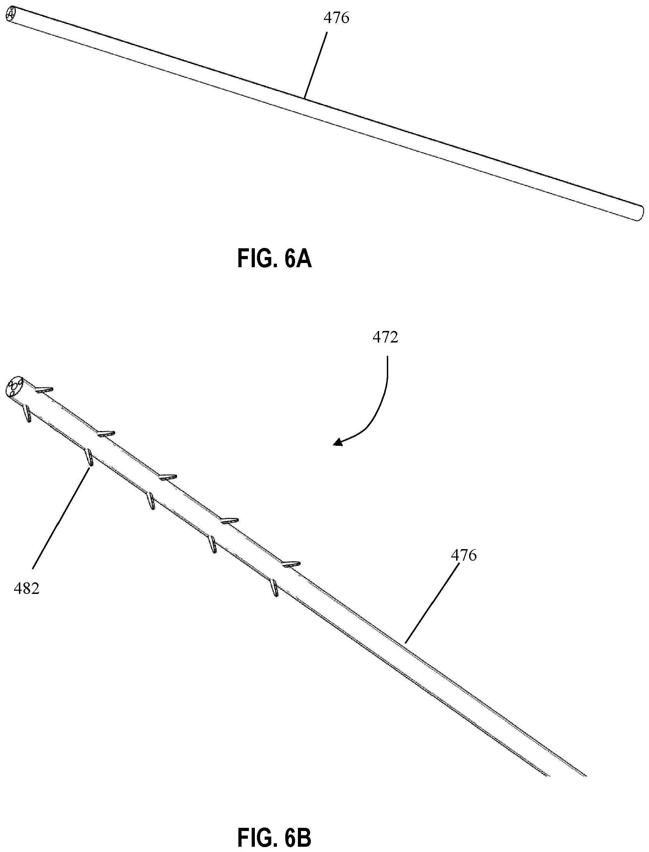

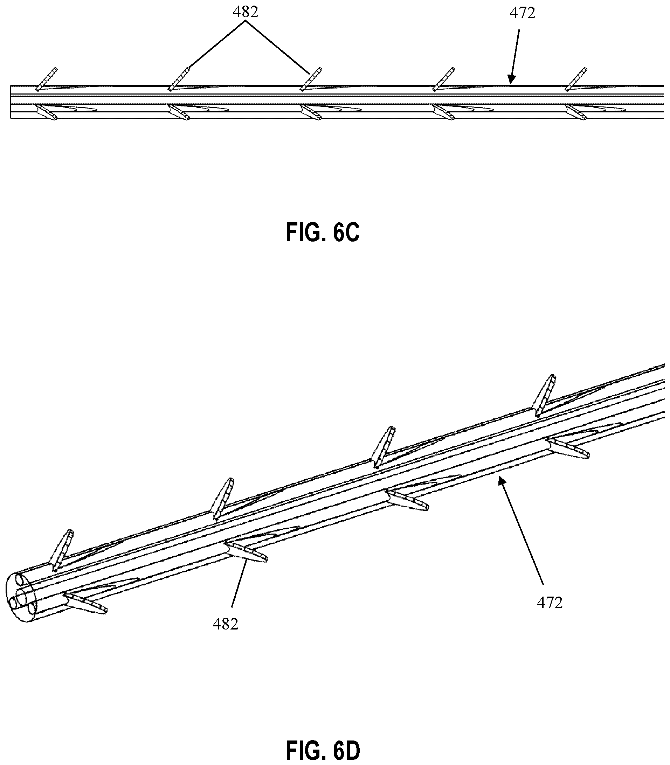

Referring now to FIGS. 6A-6D, a lead assembly 472 is depicted according to an embodiment of the disclosure. Lead assembly 472 includes a plurality of rows of fixation elements which may be included on a length of tubing 476 coupleable to a lead body, or may be included directly as part of a lead body (not shown). A plurality of rows of barbs 482 are included, each of the rows generally arranged around a circumference of the tubing or lead body. Barbs 482 are movable between a retracted position depicted in FIG. 6A and the deployed, extended position depicted in FIGS. 6B-6D. Lead assembly 472 may include a plurality of lumens, each corresponding to a row of fixation elements. The barbs 482 may extend during device assembly during the manufacturing stage, or the barbs 482 may extend as a mechanism during the delivery of the lead, after implantation. The barbs 482 may be exposed on the lead 472 as the polymer wires of the lumen design are pulled backwards after implantation or during manufacturing. The extended barbs are intended to allow movement of the lead body toward a target implant location, but not in an opposite direction. If the barbs are not deployed or extended then the lead body should be able to move both forwards and backwards, deployment of the barbs aids in fixation and acts as a fixation mechanism for the affected portion of the lead body.

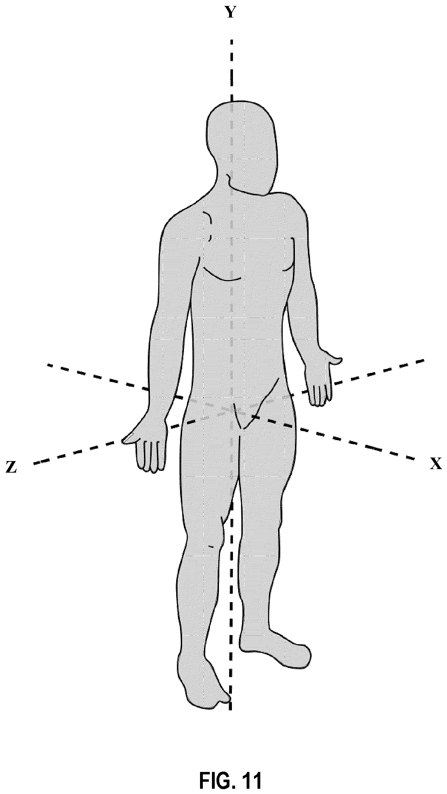

In other embodiments, aspects of the present disclosure pertain to improvements for fixation of the electrode structure in one or more directions or axes. Fixation may be of the passive type, active type, or combinations thereof. The lead body fixation mechanisms restrict movement of the lead in the axial direction, whereas the electrode fixation mechanisms fix the actual electrode in place (typically in all directions, x, y and z as depicted in FIG. 11).

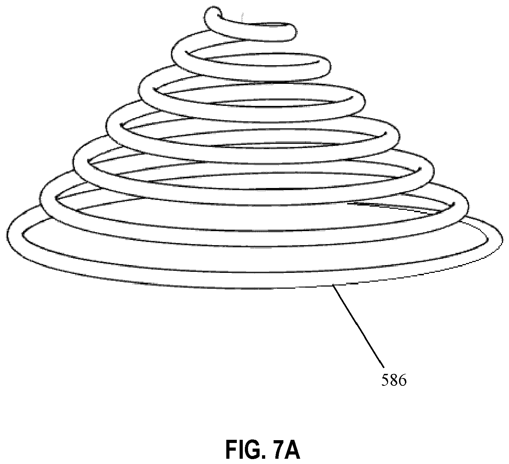

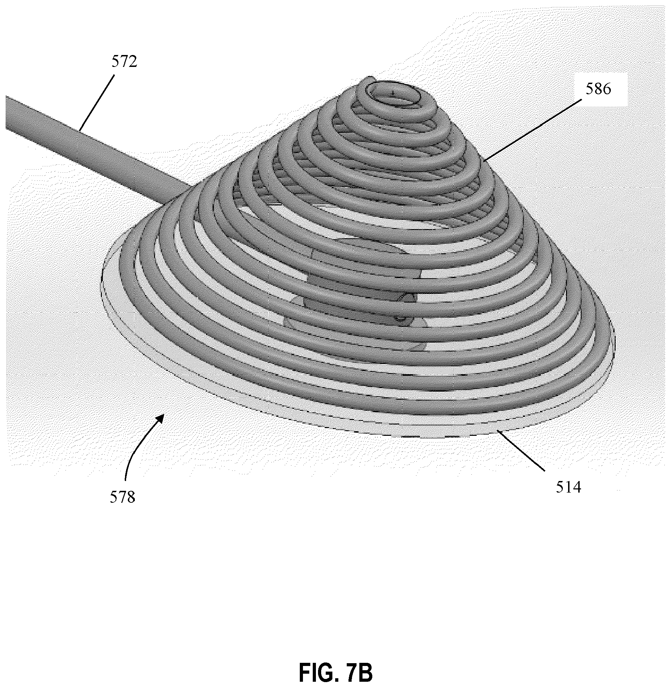

Referring now to FIGS. 7A-7B, an electrode structure 578 including a coil 586 is depicted according to an embodiment of the disclosure. The coil 586 may be molded into, or mounted on, integrated with, or otherwise coupled to a backer 514. Coil 586 may be constructed of a shape memory alloy such as nitinol, of a resorbable polymer, or of other suitable materials. The coil height, and number of revolutions may also vary as desired. Coil 586 may be biased in an extended, conical position such as depicted in FIGS. 7A-7B. It will be understood coil 586 is also compressible into a substantially collapsed position suitable for delivery to a target implant location. Electrode structure 578, including coil 586, may be combined with any of the lead assembly embodiments described herein

In use, electrode structure 578 may be arranged in a delivery position wherein coil 586 is substantially collapsed with respect to backer 514. An introducer or delivery tool may be utilized to selectively retain electrode structure 578 in the delivery position. A surgeon or medical practitioner may tunnel or otherwise dilate tissue within a patient along a desired path of introduction to a target implant location. Electrode structure 578 may be delivered concurrently with a tunneling procedure, or subsequently. Upon delivery of electrode structure 578 to the target implant location, coil 586 may be released into the deployed position depicted in FIGS. 7A-7B, such as by retraction of the introducer or delivery tool. Once in the deployed position, coil 586 expands in a lateral anatomical direction (e.g., away from a midline of the body of the patient) to bias against surrounding tissue or fill any tissue space created by the tunneling and thus helps to retain backer 514 against the target location. In the deployed position, expanded coil 586 further helps to retain backer 514 and electrode structure 578 in the superior/inferior and anterior/posterior anatomical directions, thereby preventing migration of electrode structure 578, reduced effectiveness of therapy delivered, or both.

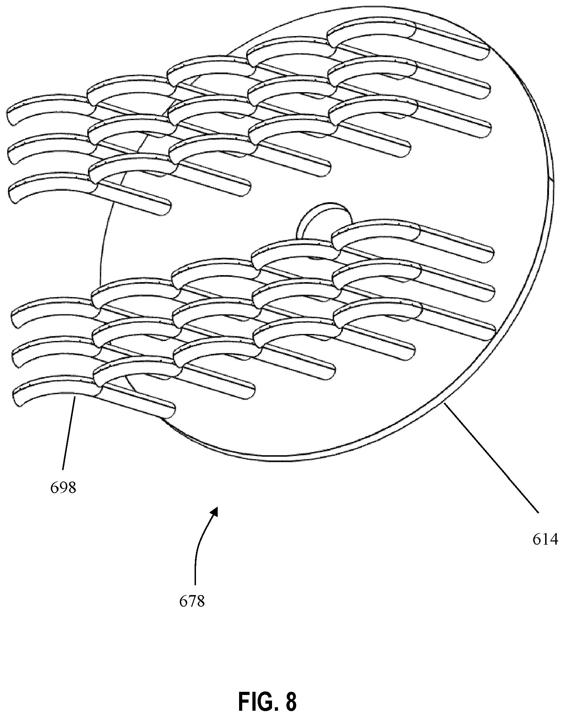

Referring now to FIG. 8, an electrode structure 678 including a plurality of hooks 698 is depicted, according to an embodiment of the disclosure. Hooks 698 may be molded into, or mounted on, integrated with, or otherwise coupled to a backer 614. Hooks 698 may be constructed of a shape memory alloy such as nitinol, of a resorbable polymer, of a rigid polymer, or of other suitable material. In embodiments, hooks 698 may be compressible or otherwise movable between a delivery position wherein hooks 698 are generally proximate backer 614, and the deployed position depicted in FIG. 8. Hooks 698 may be arranged on an inactive or back side of electrode structure 678, opposite the side on which an electrode is included. The height, number and spacing of hooks 698 may be varied as desired to optimize fixation, and aid in ease of delivery. Electrode structure 678, including hooks 698, may be combined with any lead assembly embodiments described herein.

In embodiments wherein hooks 698 are collapsible, in use, electrode structure 678 may be arranged in a delivery position wherein hooks 698 are substantially collapsed with respect to backer 614. An introducer or delivery tool may be utilized to selectively retain electrode structure 678 in the delivery position. A surgeon or medical practitioner may tunnel or otherwise dilate tissue within a patient along a desired path of introduction to a target implant location. Electrode structure 678 may be delivered concurrently with a tunneling procedure, or subsequently. Upon delivery of electrode structure 678 to the target implant location, hooks 698 may be released into the deployed position depicted in FIG. 8, such as by retraction of the introducer or delivery tool. Once in the deployed position, hooks 698 expand in a lateral anatomical direction (e.g., away from a midline of the body of the patient) to fill any tissue space created by the tunneling and thus helps to retain backer 614 against the target location. In the deployed position, expanded hooks 698 help to retain backer 614 and electrode structure 678 in the superior/inferior and anterior/posterior anatomical directions, thereby preventing migration of electrode structure 678, reduced effectiveness of therapy delivered, or both.

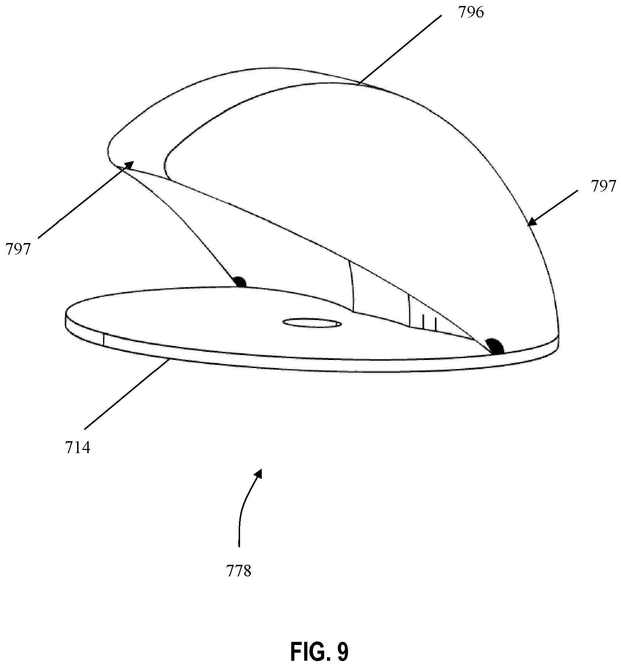

Referring now to FIG. 9 an electrode structure 778 including a hood or dome-shaped protrusion is depicted, according to an embodiment of the disclosure. The dome 796 may comprise a molded structure as depicted, or alternately constructed with a nitinol frame. Dome 796 may be molded onto, or mounted on, integrated with, or otherwise coupled to a backer 714. Dome 796 may include a leading profile 797, and a hook portion 798. Electrode structure 778, including dome 796, may be combined with any lead assembly embodiments described herein.

In use, electrode structure 778 may be implanted using an introducer or delivery tool. A surgeon or medical practitioner may tunnel or otherwise dilate tissue within a patient along a desired path of introduction to a target implant location. Electrode structure 778 may be delivered concurrently with a tunneling procedure, or subsequently. Upon delivery of electrode structure 778, dome 796 is configured to substantially fill any tissue space created by the tunneling and thus helps to retain backer 714 against the target location. Additionally, leading profile 797 constrains movement of electrode structure 778 from any further movement or migration in the direction of tunneling, while hook portion 798 constrains movement of electrode structure 778 from retraction or backward movement or migration in the direction of tunneling. Thus electrode structure 778 with dome 796 is constrained in a lateral anatomical direction, as well as in the superior/inferior and anterior/posterior anatomical directions.

In other embodiments, aspects of the present disclosure pertain to improvements for delivery tools used during the implant procedure of baroreflex activation devices.

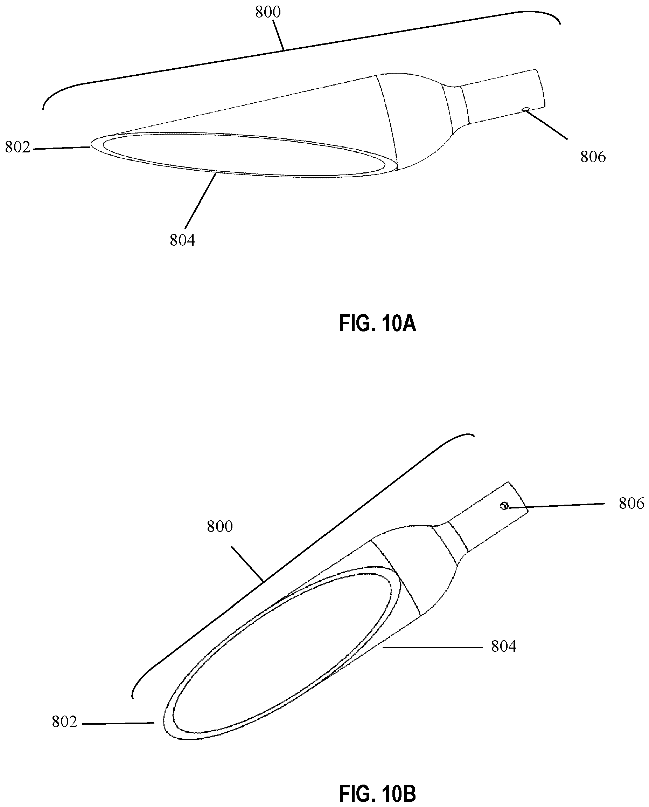

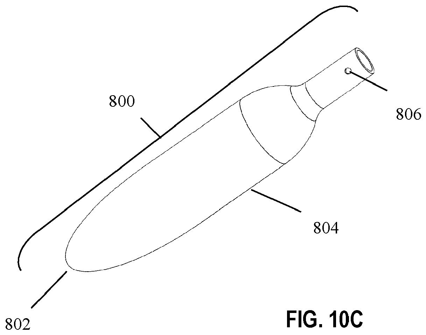

Referring now to FIGS. 10A-C, an embodiment of a needle tip for an electrode. The needle tip 800 may have a substantially pointed tip 802, a base 804, and an aperture 806 to connect the needle tip 800 to an electrode structure or lead assembly. The substantially pointed tip 802 may be designed such that tip 802 aids in the implantation delivery of treatment to a target. The needle tip 800 may retract when implantation is complete and the electrode is fixated, or the needle tip 800 may be made of a bio-resorbable material that is not removed after the implantation process.

Embodiments of devices and exemplary methods of use for introduction and implantation of an electrode structure for baroreflex activation therapy, as part of a minimally invasive implant technique, are disclosed herein. An implantable baroreflex activation system includes a control system having an implantable housing, an electrical lead, attachable to the control system, and an electrode structure. One or more of the electrical lead or electrode structure may include elements to improve fixation at a target implant location. Improvements to delivery tools associated with implant are also described.

Various embodiments of systems, devices, and methods have been described herein. These embodiments are given only by way of example and are not intended to limit the scope of the disclosure. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the disclosure.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended. The invention, however, is defined by the appended claims.

## Claims

1. An implantable baroreflex activation therapy system (90), comprising:
a control system (60) having an implantable housing (68);
an electrical lead (72), coupleable to the control housing (68); and
an electrode structure (78) proximate a distal end of the electrical lead (72), the electrode structure (78) including:
a backer (114) having a first side and a second side opposite the first side, the first side configured for implantation on an outer surface of a blood vessel;
an electrode (112), arranged on the first side, the backer (114) having an effective surface area at least twice an area of the electrode (112); and
an expandable coil (586) arranged on the second side of the backer (114), the coil (586) selectively expandable in a direction orthogonal to a face of the second side of the backer (114),
wherein the control system (60) is programmed to deliver a baroreflex therapy via the electrode (112) to a baroreceptor within a wall of the blood vessel.

2. The implantable baroreflex activation therapy system (90) of claim 1, wherein the electrode (112) comprises a cathode and the implantable housing (68) includes an anode.

3. The implantable baroreflex activation therapy system (90) of claim 1 or 2, wherein the backer (114) is electrically insulative.

4. The implantable baroreflex activation therapy system (90) of claim 1, 2 or 3, wherein the electrode structure (78) is configured such that the expandable coil (586) biases the electrode (112) into contact with the blood vessel when implanted.

5. The implantable baroreflex activation therapy system (90) of any preceding claim, wherein the expanded coil (586) is configured to retain the electrode structure (78) in the superior and inferior anatomical direction, and further retain the electrode structure (78) in the anterior and posterior anatomical direction, so as prevent migration of the electrode structure (78) subsequent to implantation.

6. The implantable baroreflex activation therapy system (90) of any preceding claim, wherein the expandable coil (586) is cone shaped in an expanded position.

7. The implantable baroreflex activation therapy system (90) of any preceding claim, further comprising a delivery sheath configured to retain the expandable coil (586) in a retracted position, the delivery sheath selectively removable to allow expansion of the coil (586).

8. The implantable baroreflex activation therapy system (90) of claim 1, wherein the electrode structure (78) is configured for tunneling from an incision in a patient toward a target implant site by dissecting tissue to create the tunnel.

9. The implantable baroreflex activation therapy system (90) of claim 8, further comprising a delivery tool configured to push the rigid electrode structure to dissect tissue to create the tunnel.

10. The implantable baroreflex activation therapy system (90) of claim 8 or 9, wherein at least one of:
the system (90) comprises a fixation element configured to be deployed on the electrode structure (78) to secure the electrode structure (72) at an implant location;
the system (90) comprises a fixation element proximate the distal end of the electrical lead (72) to secure the electrode structure (72) at an implant location; and
the electrode (112) is configured to contact an outer surface of the blood vessel to secure the electrode structure (78) at an implant location.

11. The implantable baroreflex activation therapy system (90) of any of claims 8 to 10, wherein the electrode structure (78) is configured to be secured to a blood vessel, wherein said blood vessel is selected from the group consisting of: a carotid sinus, an aortic arch, a common carotid artery, a subclavian artery, a brachiocephalic artery, and a heart.

## Patentansprüche

1. Implantierbares Baroreflex-Aktivierungstherapiesystem (90), das Folgendes umfasst:
ein Steuersystem (60) mit einem implantierbaren Gehäuse (68);
eine elektrische Leitung (72), die mit dem Steuergehäuse (68) verbunden werden kann; und
eine Elektrodenstruktur (78) in der Nähe eines distalen Endes der elektrischen Leitung (72), wobei die Elektrodenstruktur (78) Folgendes enthält:
eine Unterlage (114) mit einer ersten Seite und einer der ersten Seite gegenüberliegenden zweiten Seite, wobei die erste Seite für die Implantation auf einer Außenfläche eines Blutgefäßes konfiguriert ist;
eine Elektrode (112), die auf der ersten Seite ausgelegt ist, wobei die Unterlage (114) eine effektive Oberfläche aufweist, die mindestens doppelt so groß ist wie eine Fläche der Elektrode (112); und
eine expandierbare Spule (586), die auf der zweiten Seite der Unterlage (114) ausgelegt ist, wobei die Spule (586) selektiv in einer Richtung orthogonal zu einer Oberfläche der zweiten Seite der Unterlage (114) expandierbar ist,
wobei das Steuersystem (60) so programmiert ist, dass es eine Baroreflextherapie über die Elektrode (112) an einen Barorezeptor in einer Wand des Blutgefäßes abgibt.

2. Implantierbares Baroreflex-Aktivierungstherapiesystem (90) nach Anspruch 1, wobei die Elektrode (112) eine Kathode umfasst und das implantierbare Gehäuse (68) eine Anode enthält.

3. Implantierbares Baroreflex-Aktivierungstherapiesystem (90) nach Anspruch 1 oder 2, wobei die Unterlage (114) elektrisch isolierend ist.

4. Implantierbares Baroreflex-Aktivierungstherapiesystem (90) nach Anspruch 1, 2 oder 3, wobei die Elektrodenstruktur (78) so konfiguriert ist, dass die expandierbare Spule (586) die Elektrode (112) in Kontakt mit dem Blutgefäß vorspannt, wenn sie implantiert ist.

5. Implantierbares Baroreflex-Aktivierungstherapiesystem (90) nach einem der vorhergehenden Ansprüche, wobei die expandierte Spule (586) dafür konfiguriert ist, die Elektrodenstruktur (78) in der oberen und unteren anatomischen Richtung zu halten und die Elektrodenstruktur (78) ferner in der vorderen und hinteren anatomischen Richtung zu halten, um eine Migration der Elektrodenstruktur (78) nach der Implantation zu verhindern.

6. Implantierbares Baroreflex-Aktivierungstherapiesystem (90) nach einem der vorhergehenden Ansprüche, wobei die expandierbare Spule (586) in einer expandierten Position kegelförmig ist.

7. Implantierbares Baroreflex-Aktivierungstherapiesystem (90) nach einem der vorhergehenden Ansprüche, das ferner eine Zuführungshülle umfasst, die so konfiguriert ist, dass sie die expandierbare Spule (586) in einer zurückgezogenen Position hält, wobei die Zuführungshülle selektiv entfernbar ist, um eine Expansion der Spule (586) zu ermöglichen.

8. Implantierbares Baroreflex-Aktivierungstherapiesystem (90) nach Anspruch 1, wobei die Elektrodenstruktur (78) dafür konfiguriert ist, von einer Inzision in einem Patienten zu einer Zielimplantationsstelle zu tunneln, indem Gewebe zur Erzeugung des Tunnels seziert wird.

9. Implantierbares Baroreflex-Aktivierungstherapiesystem (90) nach Anspruch 8, das ferner ein Einführwerkzeug umfasst, das dafür konfiguriert ist, die starre Elektrodenstruktur zu schieben, um Gewebe zu sezieren und den Tunnel zu erzeugen.

10. Implantierbares Baroreflex-Aktivierungstherapiesystem (90) nach Anspruch 8 oder 9, wobei mindestens eines der Folgenden gilt:
das System (90) umfasst ein Fixierungselement, das dafür konfiguriert ist, auf der Elektrodenstruktur (78) eingesetzt zu werden, um die Elektrodenstruktur (72) an einer Implantationsstelle zu sichern;
das System (90) umfasst ein Fixierungselement in der Nähe des distalen Endes der elektrischen Leitung (72), um die Elektrodenstruktur (72) an einer Implantationsstelle zu sichern; und
die Elektrode (112) ist dafür konfiguriert, eine äußere Oberfläche des Blutgefäßes zu kontaktieren, um die Elektrodenstruktur (78) an einer Implantationsstelle zu sichern.

11. Implantierbares Baroreflex-Aktivierungstherapiesystem (90) nach einem der Ansprüche 8 bis 10, wobei die Elektrodenstruktur (78) dafür konfiguriert ist, an einem Blutgefäß befestigt zu werden, wobei das genannte Blutgefäß ausgewählt ist aus der Gruppe bestehend aus: einem Karotis-Sinus, einem Aortenbogen, einer gemeinsamen Karotisarterie, einer Subclaviaarterie, einer Brachiocephalusarterie und einem Herz.

## Revendications

1. Système de thérapie d'activation du baroréflexe implantable (90), comprenant :
un système de commande (60) ayant un logement implantable (68) ;
un fil électrique (72), pouvant être couplé au logement de commande (68) ; et
une structure d'électrode (78) à proximité d'une extrémité distale du fil électrique (72), la structure d'électrode (78) incluant :
un support (114) ayant un premier côté et un deuxième côté opposé au premier côté, le premier côté configuré pour une implantation sur une surface externe d'un vaisseau sanguin ;
une électrode (112), agencée sur le premier côté, le support (114) ayant une aire de surface active représentant au moins le double d'une aire de l'électrode (112) ; et
un enroulement capable de déploiement (586) agencé sur le deuxième côté du support (114), l'enroulement (586) capable de déploiement sélectif dans une direction orthogonale à une face du deuxième côté du support (114),
dans lequel le système de commande (60) est programmé pour délivrer une thérapie de baroréflexe via l'électrode (112) à un barorécepteur au sein d'une paroi du vaisseau sanguin.

2. Système de thérapie d'activation de baroréflexe implantable (90) selon la revendication 1, dans lequel l'électrode (112) comprend une cathode et le logement implantable (68) inclut une anode.

3. Système de thérapie d'activation de baroréflexe implantable (90) selon la revendication 1 ou 2, dans lequel le support (114) est isolant électriquement.

4. Système de thérapie d'activation de baroréflexe implantable (90) selon la revendication 1, 2 ou 3, dans lequel la structure d'électrode (78) est configurée de sorte que l'enroulement capable de déploiement (586) sollicite l'électrode (112) en contact avec le vaisseau sanguin quand elle est implantée.

5. Système de thérapie d'activation de baroréflexe implantable (90) selon l'une quelconque des revendications précédentes, dans lequel l'enroulement déployé (586) est configuré pour retenir la structure d'électrode (78) dans la direction anatomique supérieure et inférieure, et retenir en outre la structure d'électrode (78) dans la direction anatomique antérieure et postérieure, de manière à empêcher la migration de la structure d'électrode (78) suite à l'implantation.

6. Système de thérapie d'activation de baroréflexe implantable (90) selon l'une quelconque des revendications précédentes, dans lequel l'enroulement capable de déploiement (586) est en forme de cône dans une position déployée.

7. Système de thérapie d'activation de baroréflexe implantable (90) selon l'une quelconque des revendications précédentes, comprenant en outre une gaine de délivrance configurée pour retenir l'enroulement capable de déploiement (586) dans une position rétractée, la gaine de délivrance amovible sélectivement pour permettre le déploiement de l'enroulement (586).

8. Système de thérapie d'activation de baroréflexe implantable (90) selon la revendication 1, dans lequel la structure d'électrode (78) est configurée pour percer un tunnel d'une incision dans un patient à un site d'implant cible en disséquant du tissu pour créer le tunnel.

9. Système de thérapie d'activation de baroréflexe implantable (90) selon la revendication 8, comprenant en outre un outil de délivrance configuré pour pousser la structure d'électrode rigide pour disséquer du tissu pour créer le tunnel.

10. Système de thérapie d'activation de baroréflexe implantable (90) selon la revendication 8 ou 9 , dans lequel l'un parmi :
le système (90) comprend un élément de fixation configuré pour être déployé sur la structure d'électrode (78) pour fixer la structure d'électrode (72) au niveau d'un emplacement d'implant ;
le système (90) comprend un élément de fixation à proximité de l'extrémité distale du fil électrique (72) pour fixer la structure d'électrode (72) au niveau d'un emplacement d'implant ; et
l'électrode (112) est configurée pour entrer en contact avec une surface externe du vaisseau sanguin pour fixer la structure d'électrode (78) au niveau d'un emplacement d'implant.

11. Système de thérapie d'activation de baroréflexe implantable (90) selon l'une quelconque des revendications 8 à 10, dans lequel la structure d'électrode (78) est configurée pour être fixée à un vaisseau sanguin, dans lequel ledit vaisseau sanguin est choisi parmi le groupe constitué par : un sinus carotidien, un arc aortique, une artère carotide commune, une artère subclavière, une artère brachiocéphalique, et un coeur.
